# EUROPÄISCHE PATENTSCHRIFT

(11) **EP 0 496 222 B1**
(45) Veröffentlichungstag und Bekanntmachung des Hinweises auf die Patenterteilung: **09.09.1998**
(21) Anmeldenummer: 92100343.0
(22) Anmeldetag: 10.01.1992
(51) Int. Cl.: C07D 209/14, A61K 31/40, C07D 401/04, C07D 401/06, C07D 403/06, C07D 498/10, C07D 405/12

(54) **Indolderivate**
Indole derivatives
Dérivés de l'indole

(30) Priorität: 22.01.1991 DE 4101686
(43) Veröffentlichungstag der Anmeldung: 29.07.1992
(73) Patentinhaber: MERCK PATENT GmbH, 64271 Darmstadt (DE)
(72) Erfinder: Böttcher, Henning, Dr., W-6100 Darmstadt (DE); Greiner, Hartmut, Dr., W-6100 Darmstadt (DE); Seyfried, Christoph, Dr., W-6104 Jugenheim (DE); Bartoszyk, Gerd, W-6100 Darmstadt (DE)

(56) Entgegenhaltungen:
- EP-A- 0 376 607
- EP-A- 0 407 844
- BE-A- 771 285
- FR-A- 1 551 082
- GB-A- 1 075 156
- GB-A- 1 189 064
- JOURNAL OF PHARMACEUTICAL SCIENCES Bd. 62, Nr. 3, März 1973, Seiten 508 - 509; B. T. HO ET AL.: 'Inhibitors of Hydroxyindole-O-methyltransferase: Indolealkylpiperazines'
- CHEMICAL ABSTRACTS, vol. 78, no. 9, 5. März 1973, Columbus, Ohio, US; abstract no. 58465G, SUMITOMO CHEMICAL CO., LTD.: 'Indole derivatives' Seite 527 ;Spalte 1 ;

## Beschreibung

Die Erfindung betrifft neue Indolderivate der Formel I 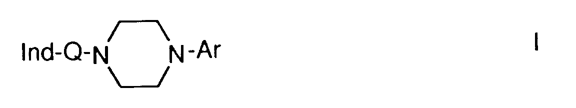 worin
- Ind: einen durch CN, CO-R¹, CO-NR³R⁴ oder NHR² substituierten Indol-3-yl-rest,
- R¹: NH₂, NHA, NA₂, NHCₙH₂ₙNA₂, NHCₙH₂ₙHet oder NHCₙH₂ₙOA,
- R²: CO-NH₂, CO-NHA oder CO-NA₂,
- R³ und R⁴: zusammen eine Alkylengruppe mit 3-7 C-Atomen, die durch O oder NR⁵ unterbrochen und/oder durch O, NA₂, NHCOA, COOA, CONH₂, Ar oder Het substituiert sein und/oder eine zusätzliche Doppelbindung enthalten kann,
- R⁵: H, A, Ar, Het, Ar-CO, COOA, CH₂CONH₂, CH₂CONHA, CH₂CONA₂ oder CHO,
- Q: C₄H₈,
- n: 1, 2, 3, 4, 5 oder 6,
- A: Alkyl mit 1-6 C-Atomen,
- Ar: einen unsubstituierten oder einen ein-, zwei- oder dreifach durch A, F, Cl, Br, I, CN, OH, OA, NHCOA und/oder CF₃ oder durch eine Methylendioxygruppe substituierten Phenylrest und
- Het: einen gesättigten oder ungesättigten 5- oder 6-gliedrigen heterocyclischen Rest mit 1-4 N-, O-und/oder S-Atomen, der mit einem Benzolring kondensiert und/oder ein- oder zweifach durch A, Ar, -O(CH₂)₂O-, Carbonylsauerstoff oder einen weiteren Rest Het substituiert sein kann,
bedeuten
sowie deren Salze.

Der Erfindung lag die Aufgabe zugrunde, neue Verbindungen aufzufinden, die zur Herstellung von Arzneimitteln verwendet werden können.

Ähnliche Verbindungen sind bekannt, z.B. aus EP-A-0 407 844, EP-A-0 376 607, BE-A-771285, GB-A-1,075,156, GB-A-1,189,064 oder FR-A-1,551,082.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N.raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich als Prophylaxe und zur Bekämpfung der Folgen cerebraler Infarktgeschehen (Apoplexia cerebri), wie Schlaganfall und cerebraler Ischämien.

Verbindungen der Forniel I und ihre physiologisch unbedenklichen Säureadditionssalze können daher als Arzneimittelwirkstoffe für Anxiolytika, Antidepressiva, Neuroleptika und/oder Antihypertonika und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. NHA ist vorzugsweise Methylamino, ferner Ethylamino, n-Propylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino.

Analog bedeutet CO-NHA vorzugsweise N-Methylcarbamoyl oder N-Ethylcarbamoyl; CO-NA₂ vorzugsweise N,N-Dimethylcarbamoyl oder N,N-Diethylcarbamoyl.

Der Rest Ar bedeutet vorzugsweise unsubstituiertes Phenyl, aber auch ein-, zwei- oder dreifach substituiertes Phenyl. Falls der Phenylrest zweifach substituiert ist, können die Substituenten gleich oder verschieden sein. Bevorzugte Substituenten an der Phenylgruppe sind F, Cl, Methoxy, CN, CF₃, NHCOCH₃ oder Methyl. Die Substituenten befinden sich im Fall der substituierten Phenylreste in ortho-, meta- und/oder para-Position, wobei zwei- und dreifach substituierte Phenylreste bevorzugt ortho- und parasubstituiert sind. Im einzelnen ist Ar bevorzugt Phenyl, o-, m- oder p-Trifluormethylphenyl, o-, m- oder p-Methoxyphenyl, o-, m- oder p-Fluorphenyl, o-, m- oder p-Methylphenyl, o-, m- oder p-Cyanphenyl oder 2,4-Dimethoxyphenyl, aber auch o-, m- oder p-Ethoxyphenyl, o-, m- oder p-Bromphenyl, 2,3-, 2,5-, 2,6-, 3,4- oder 3,5-Dimethoxyphenyl, 2,3- oder 3,4-Methylendioxyphenyl sowie 3,5-Dichlor-4-methoxyphenyl.

Het ist vorzugsweise 1-Pyrrolidinyl-, 1-Piperidinyl, 1,2- Dihydro-1-pyridinyl, 1,2,3,6-Tetrahydro-1-pyridinyl, 4-Morpholinyl oder 1-Piperazinyl, ferner auch 2,6-, 2,5-, 3,5- oder 3,6-Dimethyl-4-morpholinyl.

Het ist außerdem vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6-, 7- oder 8-Chinazolyl. Weitere heterocyclische Reste, die teilweise oder vollständig hydriert sind, können z. B. auch bedeuten 2,3-Dihydro-2-, -3-, -4- oder - 5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2- oder 3-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl.

Die heterocyclischen Reste können auch wie angegeben substituiert sein. Het kann z.B. auch bedeuten: 4- oder 5-Methyl-2-thiazolyl, 4-, 5- oder 6-Methyl-2-pyrimidinyl, 4,5-Dimethyl-2-thiazolyl, 3-, 4- oder 5-Methyl-2-furyl, 2-, 4- oder 5-Methyl-3-furyl, 2,4-Dimethyl-3-furyl, 3-, 4- oder 5-Methyl-2-thienyl, 2-, 4- oder 5-Methyl-3-thienyl, 3-Methyl-5-tert.-butyl-2- thienyl, ferner auch z.B. 2-, 3- oder 4-Phenyl-1-piperidinyl, 2-, 3-, 5- oder 6-Phenyl-1-morpholinyl.

Der Rest Ind bedeutet einen einfach durch einen der angegebenen Reste substituierten Indol-3-ylrest. Vorzugsweise ist er in 5-Stellung, ferner auch in der 4-, 6- oder 7-Stellung substituiert. Weiterhin ist eine Substitution in 1- oder 2-Stellung möglich. Bevorzugte Substituenten am Indol-3-ylrest sind CN, CONH₂ oder H₂N-CO-NH.

Der Parametern kann 1, 2, 3, 4, 5 oder 6 sein, vorzugsweise ist er 1, 2 oder 4.

Der Rest Q ist vorzugsweise -(CH₂)₄-.

R¹ ist bevorzugt CN.

R² ist vorzugsweise CO-NH₂, ferner CO-NH-CH₃ oder CO-N(CH₃)₂.

R³ und R⁴ treten immer in Form von -NR³R⁴ auf. Die Gruppe -NR³R⁴ ist vorzugsweise 1-Piperidinyl, 4-R⁵-Pipendinyl, 1,2-Dihydro-1-pyridinyl, 1,2,3,6-Tetrahydro-1-pyridinyl, 4-Morpholinyl, 1-Piperazinyl, 3-Keto-1-piperazinyl, 4-R⁵-1-Piperazinyl oder 1-Pyrrolidino, ferner auch 2,6-, 2,5- oder 3,5-Dimethyl-4-morpholinyl.

R⁵ ist vorzugsweise H, A, Ar, 2-Pyrimidinyl, 4- oder 5-Methyl-2-thiazol, Ar-C0, COOA, CH₂CONHA oder CHO.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ie ausgedrückt werden, die der Formel I entsprechen und worin die nicht näher bezeichneten Reste und Parameter die bei der Formel I angegebene Bedeutung haben, worin jedoch
- in Ia: Ind einen in 5-Stellung durch CO-R¹ substituierten Indol-3-yl-rest bedeutet;
- in Ib: Ind einen in 5-Stellung durch NHR² substituierten Indol-3-yl-rest bedeutet;
- In Ic: Ind einen in 5-Stellung durch CONH₂ substituierten Indol-3-yl-rest bedeutet;
- In Id: Ind einen in 5-Stellung durch CN substituierten Indol-3-yl-rest bedeutet;
- in Ie: Ind einen in 5-Stellung durch NH-CO-NH₂ substituierten Indol-3-yl-rest bedeutet.

Insbesondere sind bevorzugt Verbindungen der Teilformeln If sowie Iaf bis Ief, die den Teilformeln I sowie Ia bis Ie entsprechen, worin jedoch zusätzlich
- Q: -(CH₂)₄-
bedeutet.

Ferner sind insbesondere bevorzugt Verbindungen der Teilformeln Ig sowie Iag bis Ieg, die den Teilformeln I sowie Ia bis Ie entsprechen, worin jedoch zusätzlich
- Ar: Phenyl, o-, m- oder p-Methoxyphenyl, 2,4-Dimethoxyphenyl, o-, m- oder p-Fluorphenyl oder aber o-, m- oder p-Cyanphenyl
bedeutet.

Gegenstand der Erfindung ist ferner ein Verfahren zur Herstellung von Indolderivaten der Formel I sowie von deren Salzen, dadurch gekennzeichnet, daß man eine Verbindung der Formel II

Ind-Q-X¹ II

worin
X¹ X oder NH und
X Cl, Br, I, OH oder eine reaktionsfähig funktionell abgewandelte OH-Gruppe bedeuten und
Ind und Q die angegebenen Bedeutungen haben, mit einer Verbindung der Formel III

   X²-CH₂-CH₂-NAr-CH₂-CH₂X³ III

   worin
X² und X³ gleich oder verschieden sein können und, falls X¹ = NH₂ ist, jeweils X, andernfalls zusammen NH bedeuten und
Ar die angegebene Bedeutung hat,
   umsetzt
   oder daß man eine Verbindung der Formel IV

   Ind-Q-N (CH₂-CH₂-X)₂ IV IV

   worin
X, Q und Ind die angegebenen Bedeutungen haben, mit einer Verbindung der Formel V

   Ar-NH₂ V

   worin
Ar die angegebene Bedeutung hat,
   umsetzt
   oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt
   oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
   und/oder daß man gegebenenfalls eine OA-Gruppe unter Bildung einer OH-Gruppe spaltet und/oder eine Gruppe Ind und/oder eine Gruppe Ar in eine andere Gruppe Ind und/oder Ar umwandelt und/oder daß man eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 33 42 632) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist X¹ vorzugsweise X; dementsprechend sind in den Verbindungen der Formel III X² und X³ vorzugsweise zusammen NH. Der Rest X ist vorzugsweise Cl oder Br; er kann jedoch auch I, OH oder eine reaktionsfähig funktionell abgewandelte OH-Gruppe bedeuten, insbesondere Alkylsulfonyloxy mit 1-6 (z. B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von Verbindungen der Formel Ind-Q-Cl oder Ind-Q-Br mit Piperazinderivaten der Formel III, worin X² und X³ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden.

Primäre Alkohole der Formel Ind-Q-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-Q-Hal. Die entsprechenden Sulfonyloxyverbindungen sind erhältlich aus den Alkoholen Ind-Q-OH durch Umsetzung mit den entsprechenden Sulfonsäurechloriden.

Die Iodverbindungen der Formel Ind-Q-I sind z. B. durch Einwirkung von Kaliumiodid auf die zugehörigen p-Toluolsulfonsäureester erhältlich. Die Amine der Formel Ind-Q-NH₂ sind z. B. aus den Halogeniden mit Phthalimidkalium oder durch Reduktion der entsprechenden Nitrile herstellbar.

Die Piperazinderivate IIIa sind größtenteils bekannt und z.B. erhältlich durch Umsetzung von Di-(2-chlorethyl)-amin mit Anilin oder einem entsprechenden, am Phenylring substituierten Derivat des Anilins. Verbindungen der Formel III (X² und X³ = jeweils X) sind z.B. herstellbar durch Reduktion von Diestern der Formel AlkylOOC-CH₂-NAr-CH₂-COOalkyl zu Verbindungen der Formel HO-CH₂-CH₂-NAr-CH₂-CH₂OH (III, X² = X³ = OH) und gegebenenfalls anschließende Umsetzung mit S0Cl₂ bzw. PBr₃.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ind-Q-NH₂ bzw. des Piperazinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Ferner ist es möglich, eine Verbindung der Formel I zu erhalten, indem man eine Verbindung der Formel Ind-Q-N(CH₂-CH₂-X)₂ (IV) mit einer Verbindung der Formel Ar-NH₂ (V) umsetzt.

Die Verbindungen der Formeln IV und insbesondere V sind zum Teil bekannt; die nicht bekannten Verbindungen können leicht in Analogie zu den bekannten hergestellt werden. So lassen sich Verbindungen der Formel IV leicht durch Umsetzung von Ind-Q-NH₂ mit 1,2-Dihalogenethan, wobei Halogen bevorzugt für Chlor oder Brom steht, herstellen. Ebenso ist es möglich, Verbindungen des Typs IV durch Umsetzung von Ind-Q-Cl, Ind-Q-Br oder Ind-Q-I mit sekundären Aminen der Formel HN(CH₂-CH₂-X)₂ zu erhalten.

Die primären Amine der Formel V lassen sich ausgehend von Anilin durch die diversen, an sich bekannten Möglichkeiten der elektrophilen Substitution am Aromaten herstellen. Ferner ist es möglich, entsprechend substituierte Nitroverbindungen durch Reduktion in die Amine der Formel V zu überführen.

Die Umsetzung der Verbindungen IV und V verläuft nach Methoden, wie sie für die Alkylierung von Aminen aus der Literatur bekannt sind. Die Komponenten können direkt, ohne Gegenwart eines Lösungsmittels, miteinander verschmolzen werden, gegebenenfalls im geschlossenen Rohr oder im Autoklaven, unter Normaldruck oder unter erhöhtem Druck, wobei ein Inertgas wie z.B. N₂ zur Druckerhöhung zugeführt wird. Es ist aber auch möglich, die Verbindungen in Gegenwart eines inerten Lösungsmittels umzusetzen. Als Lösungsmittel eignen sich die zuvor bei der Umsetzung von II mit III genannten. Ebenso kann sich der Zusatz eines säurebindenden Mittels zur Reaktionsmischung begünstigend auswirken. Es kommen die gleichen Basen, wie zuvor bei der Umsetzung der Verbindungen II und III beschrieben, in Frage.

Die optimale Reaktionszeit liegt, je nach den gewählten Reaktionsbedingungen, zwischen einigen Minuten und 14 Tagen, die Reaktionstemperatur zwischen etwa 0° und 150°, üblicherweise zwischen 20° und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder 0-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe Ind, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Bevorzugte Ausgangsstoffe für die Reduktion entsprechen der Formel VI 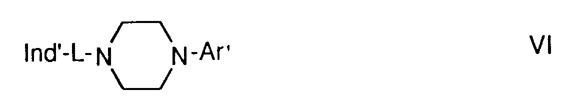 worin
- Ind': einen Rest Ind, der zusätzlich durch eine Arylsulfonylgruppe oder eine Benzylgruppe in 1-Stellung substituiert sein kann,
- L: Q oder eine dem Rest Q entsprechende Kette, worin jedoch eine oder mehrere -CH₂-Gruppe(n) durch -CO- und/oder ein oder mehrere Wasserstoffatome durch Cl, Br, F, SH- oder OH-Gruppen ersetzt sind,
- Ar': eine unsubstituierte oder ein-, zwei- oder dreifach durch A, F, Cl, Br, I, CN, OA, OH, CF₃, NHCOA und/oder O-Benzyl oder durch eine Methylendioxygruppe substituierte Phenylgruppe bedeuten,
worin jedoch nicht gleichzeitig Ind' = Ind, L = Q und Ar' = Ar sein können.

In den Verbindungen der Formel VI ist L bevorzugt -CO-(CH₂)ₙ₋₂-CO-[im einzelnen -COCO-, -COCH₂CO-, -CO-(CH₂)₂-CO-, -CO-(CH₂)₃-CO-], -(CH₂)ₙ₋₁-CO- [im einzelnen -CH₂-CO-, -CH₂CH₂-CO-, -(CH₂)₃-CO- oder -(CH₂)₄-CO-], ferner z.B. -CO-CH₂CH₂-, -CO-(CH₂)₃-, -CH₂-CO-CH₂CH₂-, -CH₂CH₂-CO-CH₂-, -CO-(CH₂)₄-, -CH₂-CO-(CH₂)₃-, -CH₂CH₂-CO-CH₂CH₂- oder -(CH₂)₃-CO-CH₂-

Verbindungen der Formel VI sind z.B. herstellbar durch Umsetzung von 4-Ar'-piperazin mit einer Verbindung der Formel VII

Ind'-L-X¹ VII

worin
Ar', Ind', L und X¹ die oben angegebenen Bedeutungen haben, unter den Bedingungen, die zuvor für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalischwässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, HaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder Li Br. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel VI, worin L eine -(CH₂)ₙ₋₁-CO-Gruppe bedeutet) mit LiAlH₄ in THF bei Temperaturen zwischen etwa 0 und 66° zu CH₂-Gruppen reduzieren. Dabei können in 1-Stellung des Indolring befindliche Arylsulfonyl-Schutzgruppen gleichzeitig reduktiv abgespalten werden. N-Benzylgruppen können reduktiv mit Natrium in flüssigem Ammoniak abgespalten werden.

Es ist ferner möglich, eine oder mehrere Carbonylgruppen nach der Methode von Wolff-Kishner zu CH₂-Gruppen zu reduzieren, z. B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Darüber hinaus ist es möglich, bestimmte Reduktionen durch Verwendung von H₂-Gas unter katalytischer Wirkung von Übergangsmetallen, wie z. B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II (X¹ = X) entsprechen, aber anstelle eines oder mehrerer H-Atome eine oder mehrere solvolysierbare Gruppe(n) enthalten. So können insbesondere 1-Acylindolderivate (entsprechend der Formel I, aber in 1-Stellung des Ind-Rests eine Acylgruppe enthaltend, vorzugsweise eine Alkanoyl-, Alkylsulfonyl- oder Arylsulfonylgruppe mit jeweils bis zu 10 C-Atomen,wie Methan-, Benzol- oder p-Toluolsulfonyl) zu den entsprechenden in der 1-Stellung des Indolringes unsubstituierten Indolderivaten hydrolysiert werden, z.B.in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel I nach an sich bekannten Methoden in eine andere Verbindung der Forniel I umwandeln.

Verbindungen der Formel I, worin Ind einen durch CO-R¹ substituierten Indol-3-yl-rest bedeutet, können durch Derivatisierung entsprechender Carboxy-indol-3-yl-Verbindungen erhalten werden. Man kann z.B. die Säuren oder ihre reaktionsfähigen Derivate, wie z.B. ihre Säurehalogenide oder Anhydride mit entsprechenden Alkoholen oder Alkoholaten, unter Verwendung der an sich bekannten Methodik oder einer der zahlreichen Varianten, verestern. Ferner ist es möglich, Säuren, Säurehalogenide, Anhydride oder Ester mit primären oder sekundären, aliphatischen oder cyclischen Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-DimethyIaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Weiterhin kann man cyan-substituierte Indol-3-yl-reste zu Carboxy-indol-3-yl- oder Carbamido-indol-3-ylresten hydrolysieren.

Verbindungen der Formel I können ferner durch Umwandlungen am Rest Ar in andere Derivate der Formel I umgewandelt werden.

Ether der Formel I, in denen der Rest Ar ein- oder zweifach durch 0-Alkyl substituiert ist, können gespalten werden, wobei die entsprechenden Hydroxyderivate entstehen. Z.B. kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilinhydrohalogeniden, vorzugsweise Pyridinhydrochlorid, bei etwa 150-250°.

Die Reste Ar können, sofern Nebenreaktionen am Indolsystem auszuschließen sind, unter den Bedingungen der Friedel-Crafts-Reaktionen chloriert, bromiert oder alkyliert werden, indem man das entsprechende Halogen oder Alkylchlorid bzw. Alkylbromid unter Katalyse von Lewis-Säuren, wie z.B. AlCl₃, FeBr₃ oder Fe, bei Temperaturen zwischen 30° und 150°, zweckmäßig zwischen 50° und 150° in einem inerten Lösungsmittel, wie z.B. Kohlenwasserstoffen, THF oder Tetrachlorkohlenstoff, mit der zu derivatisierenden Verbindung der Formel I umsetzt.

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäuren, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyciische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure` Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mglkg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Rf-Werte wurden dünnschichtchromatographisch an Kieselgel erhalten.

### Beispiel 1

Man rührt eine Lösung von 2,6 g 3-(4-Chlorbutyl)-5-indolylharnstoff [erhältlich durch Umsetzung von 5-Nitroindol mit 4-Chlorbutyrylchlorid zu 3-(4-Chlorbutyryl)-5-nitroindol, Reduktion mit Diboran zu 3-(4-Chlorbutyl)-5-nitro-indol, Hydrierung zu 3-(4-Chlorbutyl)-5-amino-indol und Umsetzung mit KCNO) und 16,3 g 1-Phenyl-piperazin ("A") in 200 ml Acetonitril 12 Std. bei 20°, arbeitet wie üblich auf und erhält 3-[4-(4-Phenyl-piperazino)-butyl]-5-indolyl-harnstoff, Hydrochlorid, F. 207° (Zers.).

### Analog erhält man:

- aus: 3-(4-Chlorbutyl)-5-cyan-indol und "A"
3-[4-(4-Phenyl-piperazino)-butyl]-5-cyan-indol, Hydrochlorid, F. 225-227°;
- aus: 3-(4-Brombutyl)-indol-5-carbonsäureamid und "A"
3-[4-(4-Phenyl-piperazino)-butyl]-indol-5-carbonsäureamid, Hydrat, Hydrochlorid, Rf 0,45 (Dichlormethan: Methanol 20:1);
- aus: 3-(4-Brombutyl)-indol-5-carbonsäuremorpholid und "A"
3-[4-(4-Phenyl-piperazino)-butyl]-indol-5-carbonsäuremorpholid;
- aus: 3-(4-Chlorbutyl)-indol-5-carbonsäurepiperidid und "A"
3-[4-(4-Phenyl-piperazino)-butyl]-indol-5-carbonsäurepiperidid;
- aus: 3-(4-Brombutyl)-indol-5-carbonsäure-4-piperidino-piperidid und "A"
3-[4-(4-Phenyl-piperazino)-butyl]-indol-5-carbonsäure-4-piperidino-piperidid;
- aus: 3-(4-Chlorbutyl)-indol-5-carbonsäure-4-morpholino-piperidid und "A"
3-[4-(4-Phenyl-piperazino)-butyl]-indol-5-carbonsäure-4-morpholino-piperidid;
- aus: 3-(4-Chlorbutyl)-indol-5-carbonsäure-4-p-chlorphenylpiperidid und "A"
3-[4-(4-Phenyl-piperazino)-butyl]-indol-5-carbonsäure-4-p-chlorphenylpiperidid;
- aus: 3-(4-Chlorbutyl)-indol-5-carbonsäure-4-N,N-dimethylamino-piperidid und "A"
3-[4-(4-Phenyl-piperazino)-butyl]-indol-5-carbonsäure-4-N,N-dimethylaminopiperidid;
- aus: 3-(4-Brombutyl)-indol-5-carbonsäure-pyrrolidid und "A"
3-[4-(4-Phenyl-piperazino)-butyl]-indol-5-carbonsäurepyrrolidid
- aus: 3-(4-Chlorbutyl)-indol-5-carbonsäure-4-methyl-piperazid und "A"
3-[4-(4-Phenyl-piperazino)-butyl]-indol-5-carbonsäure-4-methyl-piperazid.

### Beispiel 2

Analog Beispiel 1 erhält man durch Umsetzung von
3-(4-Chlorbutyl)-indol-5-carbonsäureamid mit 1-(o-Methoxyphenyl)-piperazin "B"
3-[4-(4-o-Methoxyphenyl-piperazino)-butyl]-indol-5-carbonsäureamid, Hydrat, Hydrochlorid, F. 157°.

Analog erhält man
- aus: 3-(4-Chlorbutyl)-indolyl-harnstoff und "B"
3-[4-(4-o-Methoxyphenyl-piperazino)-butyl]-indol-5-indolyl-harnstoff, Hydrochlorid, F. 130° (Zers.);
- aus: 3-(4-Chlorbutyl)-indol-5-carbonsäuremorpholid und "B"
3-[4-(4-o-Methoxyphenyl-piperazino)-butyl]-indol-5-carbonsäuremorpholid;
- aus: 3-(4-Brombutyl)-5-cyan-indol und "B"
3-[4-(4-o-Methoxyphenyl-piperazino)-butyl]-5-cyanindol;
- aus: 3-(4-Chlorbutyl)-indol-5-carbonsäure-N-methylpiperazid und 1-Phenyl-piperazin
3-[4-(4-Phenyl-piperazino)-butyl]-indol-5-carbonsäure-N-methylpiperazid;
- aus: 3-(4-Chlorbutyl)-indol-5-carbonsäure-N-p-methoxyphenyl-piperazid und 1-Phenyl-piperazin
3-[4-(4-Phenylpiperazino)-butyl]-indol-5-carbonsäure-N-p-methoxyphenylpiperazid;
- aus: 3-(4-Chlorbutyl)-indol-5-carbonsäure-3-keto-piperazid und 1-Phenyl-piperazin
3-[4-(4-Phenyl-piperazino)-butyl]-indol-5-carbonsäure-3-keto-piperazid;
- aus: 3-(4-Chlorbutyl)-indol-5-carbonsäure-N-2-pyrimidinyl-piperazid und 1-Phenyl-piperazin
3-[4-(4-Phenyl-piperazino)-butyl]-indol-5-carbonsäure-N-2-pyrimidinyl-piperazid;
- aus: 3-(4-Chlorbutyl)-indol-5-carbonsäure-N-formyl-piperazid und 1-Phenyl-piperazin
3-[4-(4-Phenyl-piperazino)-butyl]-indol-5-carbonsäure-N-formyl-piperazid;
- aus: 3-(4-Chlorbutyl)-indol-5-carbonsäure-4-ethoxycarbonyl-piperidid und 1-Phenyl-piperazin
3-[4-(4-Phenyl-piperazino)-butyl]-indol-5-carbonsäure-4-ethoxycarbonyl-piperidid;
- aus: 3-(4-Chlorbutyl)-indol-5-carbonsäure-1,2,3,6-tetrahydropyridid und 1-Phenyl-piperazin
3-[4-(4-Phenyl-piperazino)-butyl]-indol-5-carbonsäure-1,2,3,6-tetrahydropyridid.

### Beispiel 3

Ein Gemisch von 2,87 g 3-(4-Aminobutyl)-5-indolyl-harnstoff [erhältlich aus 5-Indolyl-harnstoff über 3-(4-Chlorbutyryl)-5-indolyl-harnstoff, 3-(4-Chlorbutyl)-5-indolyl-harnstoff und 3-(4-Phthalimido-butyl)-5-indolyl-harnstoff] und einem Äquivalent N,N-Bis-(2-chlorethyl)-o-cyan-anilin ("C") in 40 ml Aceton und 40 ml Wasser wird 24 Std. gekocht und wie üblich aufgearbeitet. Man erhält 3-[4-(4-o-Cyanphenyl-piperazino)-butyl]-5-indolyl-harnstoff, Hydrochlorid, F. 164-166°.

Analog erhält man
- aus: 3-(4-Aminobutyl)-5-cyan-indol und N,N-Bis-(2-chlorethyl)-p-methoxy-anilin
3-[4-(4-p-Methoxyphenyl-piperazino)butyl]-5-cyan-indol, Hydrochlorid, F. 207° (Zers.);
- aus: 3-(4-Aminobutyl)-5-cyan-indol und "C"
3-[4-(4-o-Cyanphenyl-piperazino)-butyl]-5-cyan-indol;
- aus: 3-(4-Aminobutyl)-indol-5-carbonsäureamid und N,N-Bis-(2-chlorethyl)-p-methoxy-anilin
3-[4-(4-p-Methoxyphenyl-piperazino)-butyl]-indol-5-carbonsäureamid, Hydrochlorid, F. 217° (Zers.).

### Beispiel 4

Durch Umsetzung von N,N-Dimethylcarbamoylchlorid und 3-[4-(4-p-Methoxyphenyl-piperazino)-butyl]-5-amino-indol "D" erhält man 3-[4-(4-p-Methoxyphenyl-piperazino)-butyl]-5-N,N-dimethyl-ureido-indol, Hydrochlorid, F. 187° (Zers.);
- aus: N,N-Diethylcarbamoylchlorid und "D"
3-[4-(4-p-Methoxyphenyl-piperazino)-butyl]-5-N,N-diethyl-ureido-indol, Hydrochlorid, F. 145° (Zers.).

### Beispiel 5

Eine Lösung von 3,74 g 3-[4-(4-Phenyl-piperazino)-butyl]-indol-5-carbonsäure in 500 ml DMF wird mit 1,01 g N-Methylmorpholin versetzt. Unter Rühren gibt man eine Lösung von 1 Äquivalent tert.-Butylamin in 5 ml DMF, 1,35 g 1-Hydroxybenztriazol sowie eine Lösung von 1,92 g N-(3-Dimethylaminopropyl)-N'-ethyl-carbodiimid-hydrochlorid in 20 ml DMF hinzu. Man rührt 16 Std. bei 20° und dampft das Filtrat ein. Nach üblicher Aufarbeitung erhält man 3-[4-(4-Phenyl-piperazino)-butyl]-indol-5-carbonsäure-N-tert.-butylamid.

Analog erhält man
- aus: 3-[4-(4-p-Methoxyphenyl-piperazino)-butyl]-indol-5-carbonsäure und tert.-Butylamin
3-[4-(4-p-Methoxyphenyl-piperazino)-butyl]-indol-5-carbonsäure-N-tert.-butylamid;
- aus: 3-[4-(4-p-Methoxyphenyl-piperazino)-butyl]-indol-5-carbonsäure und Morpholin
3-[4-(4-p-Methoxyphenyl-piperazino)-butyl]-indol-5-carbonsäure-morpholid, F. 112-116°;
- aus: 3-[4-(4-p-Methoxyphenyl-piperazino)-butyl]-indol-5-carbonsäure und 4-Piperidino-piperidin
3-[4-(4-p-Methoxyphenyl-piperazino)-butyl]-indol-5-carbonsäure-4-piperidino-piperidid, Hydrat, F. 116-124°;
- aus: 3-[4-(4-p-Methoxyphenyl-piperazino)-butyl]-indol-5-carbonsäure und 2,6-Dimethylmorpholin
3-[4-(4-p-Methoxyphenyl-piperazino)-butyl]-indol-5-carbonsäure-2,6-dimethyl-morpholid, Hydrat, F. 115-120°.

### Beispiel 6

Ein Gemisch von 4,05 g 3-[4-(4-p-Methoxyphenyl-piperazino)-butyl]-5-cyan-indol, 3,5 g Pyridinhydrochlorid und 80 ml Pyridin wird 3 Std. gekocht. Man kühlt ab, dampft ein, arbeitet wie üblich auf und erhält 3-[4-(4-p-Hydroxyphenyl-piperazino)-butyl]-5-cyan-indol.

### Beispiel 7

Eine Mischung von 30,6 g 3-[4-(4-m-Methoxyphenyl-piperazino)-butyl]-5-cyan-indol, 27,1 g NaOH, 520 ml Wasser und 420 ml Diethylenglykolmonoethylether wird 3 Std. bei 140° Badtemperatur gerührt. Man kühlt ab, arbeitet wie üblich auf und erhält 3-[4-(4-m-Methoxyphenyl-piperazino)-butyl]-indol-5-carbonsäureamid.

Analog erhält man durch partielle Hydrolyse der entsprechenden Nitrile:
3-[4-(4-o-Methoxyphenyl-piperazino)-butyl]-indol-5-carbonsäureamid;
3-[4-(4-Phenyl-piperazino)-butyl]-indol-5-carbonsäureamid;
3-[4-(4-p-Methoxyphenyl-piperazino)-butyl-indol-5-carbonsäureamid, F. 217° (Zers.);
3-[4-(4-o-Fluorphenyl-piperazino)-butyl]-indol-5-carbonsäureamid;
3-[4-(4-m-Fluorphenyl-piperazino)-butyl]-indol-5-carbonsäureamid;
3-[4-(4-p-Fluorphenyl-piperazino)-butyl]-indol-5-carbonsäureamid;
3-[4-(4-p-Trifluormethylphenyl-piperazino)-butyl]-indol-5-carbonsäureamid.

### Beispiel 8

Analog Beispiel 1 erhält man durch Umsetzung von 17,8 g 3-(4-Chlorbutyl)-5-indolyl-harnstoff mit einem Äquivalent 1-(o-Cyanphenyl)-piperazin 3-[4-(4-o-Cyanphenyl-piperazino)-butyl]-5-indolyl-harnstoff, F. 220-222°.

### Beispiel 9

Analog Beispiel 1 erhält man durch Umsetzung von 9,6 g 3-(4-Chlorbutyl)-5-indolyl-harnstoff mit 1 Äquivalent 1-(p-Methoxyphenyl)-piperazin 3-[4-(4-p-Methoxyphenyl)-piperazino)-butyl]-5-indolyl-harnstoff, F. 225° (Zers.).

### Beispiel 10

Man rührt eine Lösung von 10,8 g 3-[4-(N,N-Bis-(2-chlorethyl)-amino)-butyl-5-cyan-indol ("E") [erhältlich durch Umsetzung von 3-(4-Chlorbutyl)-5-cyan-indol mit N,N-Bis-(2-chlorethyl)-amin] und einem Äquivalent p-Methoxy-anilin in 200 ml Acetonitril 12 Std. bei Raumtemperatur, arbeitet wie üblich auf und erhält 3-[4-(4-p-Methoxyphenyl-piperazino)-butyl]-5-cyan-indol, F. 207° (Zers.).

Analog erhält man durch Umsetzung
- von "E: " mit o-Methoxyanilin
3-[4-(4-o-Methoxyphenyl-piperazino)-butyl]-5-cyan-indol;
- von "E": mit 2,4-Dimethylanilin
3-[4-(4-(2,4-Dimethylphenyl-piperazino)-butyl]-5-cyan-indol;
- von "E": mit p-Fluoranilin
3-[4-(4-p-Fluorphenyl-piperazino)-butyl]-5-cyan-indol;
- von "E": mit o-Fluoranilin
3-[4-(4-o-Fluorphenyl-piperazino)-butyl]-5-cyan-indol;
- von "E": mit m-Methoxyanilin
3-[4-(4-m-Methoxyphenyl-piperazino)-butyl]-5-cyan-indol;
- von "E": mit p-Trifluormethylanilin
3-[4-(4-p-Trifluormethylphenyl-piperazino)-butyl]-5-cyan-indol;
- von "E": mit 2,4-Dimethoxyanilin
3-[4-(4-(2,4-Dimethoxyphenyl)-piperazino)-butyl]-5-cyan-indol.

### Beispiel 11

Ein Gemisch von 4 g 3-[4-(4-p-Methoxyphenyl-piperazino)-butyl]-indol-5-carbonsäure, 3,2 g Pyridinhydrochlorid und 80 ml Pyridin wird 3 Std. gekocht. Man kühlt ab, dampft ein, arbeitet wie üblich auf und erhält 3-[4-(4-p-Hydroxyphenyl-piperazino)-butyl]-indol-5-carbonsäure.

Analog erhält man
- aus: 3-[4-(4-(2,4-Dimethoxyphenyl)-piperazino)-butyl]-5-cyan-indol
3-[4-(4-(2,4-Dihydroxyphenyl)-piperazino)-butyl]-5-cyan-indol.

### Beispiel 12

Man kocht 4,6 g 1-Benzolsulfonyl-3-[4-(4-phenyl-piperazino)-butyl]-5-indolyl-harnstoff [erhältlich aus 1-Benzolsulfonyl-3-(4-chlorbutyl)-5-indolyl-harnstoff und 1-Phenyl-piperazin] mit 1,5 g KOH in wässeriger Ethanollösung 16 Std., arbeitet wie üblich auf und erhält 3-[4-(4-Phenylpiperazino)-butyl]-5-indolyl-harnstoff, Hydrochlorid, F. 207° (Zers.).

### Beispiel 13

2,4 g 3-[4-(4-p-Benzyloxyphenyl-piperazino)-butyl]-indol-5-carbonsäureamid werden in 30 ml Toluol gelöst und bei Raumtemperatur 1 Std. mit H₂-Gas (p = 1 atm) unter katalytischer Wirkung von 200 mg Pd/C (Pd-Gehalt 1 %) behandelt. Anschließend filtriert man die Reaktionsmischung und erhält nach üblicher Aufarbeitung 3-[4-(4-p-Hydroxyphenylpiperazino)-butyl]-indol-5-carbonsäureamid.

### Beispiel 14

Analog Beispiel 1 erhält man durch Umsetzung von 1-(4-Methoxyphenyl)-piperazin
- mit: 3-(4-Chlorbutyl)-indol-5-carbonsäure-4-(p-methoxyphenyl)-piperazid:
3-[4-(4-(4-Methoxyphenyl)-piperazino)-butyl]-indol-5-carbonsäure-4-(p-methoxyphenyl)-piperazid, F. 146-148°;
- mit: 3-(4-Chlorbutyl)-indol-5-carbonsäure-4-methyl-piperazid:
3-[4-(4-(4-Methoxyphenyl)-piperazino)-butyl]-indol-5-carbonsäure-4-methyl-piperazid, F. 92-94°;
- mit: 3-(4-Chlorbutyl)-indol-5-carbonsäure-N-(4-methylpyridin)-amid:
3-[4-(4-(4-Methoxyphenyl)-piperazino)-butyl]-indol-5-carbonsäure-N-(4-methyl-pyridin)-amid, F. 180-182°;
- mit: 3-(4-Chlorbutyl)-indol-5-carbonsäure-3-oxo-piprazid:
3-[4-(4-(4-Methoxyphenyl)-piperazino)-butyl]-indol-5-carbonsäure-3-oxo-piperazid, F. 162-164°;
- mit: 3-(4-Chlorbutyl)-indol-5-carbonsäure-4-formyl-piperazid:
3-[4-(4-(4-Methoxyphenyl)-piperazino)-butyl]-indol-5-carbonsäure-4-formyl-piperazid, F. 159-162°;
- mit: 3-(4-Chlorbutyl)-indol-5-carbonsäure-N-(2-methoxyethyl)-amid:
3-[4-(4-(4-Methoxyphenyl)-piperazino)-butyl]-indol-5-carbonsäure-N-(2-methoxyethyl)-amid, F. 187-190°;
- mit: 3-(4-Chlorbutyl)-indol-5-carbonsäure-N-(2-piperidino-ethyl)-amid:
3-[4-(4-(4-Methoxyphenyl)-piperazino)-butyl]-indol-5-carbonsäure-N-(2-piperidino-ethyl)-amid, F. 219-221°;
- mit: 3-(4-Chlorbutyl)-indol-5-carbonsäure-N-(2-pyrrolidino-ethyl)-amid:
3-[4-(4-(4-Methoxyphenyl)-piperazino)-butyl]-indol-5-carbonsäure-N-(2-pyrrolidino-ethyl)-amid, F. 180-184°;
- mit: 3-(4-Chlorbutyl)-indol-5-carbonsäure-N-(2-(N,N-di-ethylamino)-ethyl)-amid:
3-[4-(4-(4-Methoxyphenyl)-piperazino)-butyl]-indol-5-carbonsäure-N-(2-(N,N-di-ethylamino)-ethyl)-amid, F. 221-225°;
- mit: 3-(4-Chlorbutyl)-indol-5-carbonsäure-(4-di-oxo-ethylen)-piperidid:
3-[4-(4-(4-Methoxyphenyl)-piperazino)-butyl]-indol-5-carbonsäure-(4-di-oxo-ethylen)-piperidid, F. 120-131°.

### Beispiel 15

Analog Beispiel 7 erhält man durch partielle Hydrolyse der entsprechenden Nitrile:
3-[4-(4-(3,4-Methylendioxy-phenyl)-piperazino)-butyl]-indol-5-carbonsäure-amid, F. 177-179°;
3-[4-(4-(3,5-Dichlor-4-methoxy-phenyl)-piperazino)-butyl]-indol-5-carbonsäureamid, F. 115° (Z);
3-[4-(4-(4-Hydroxy-phenyl)-piperazino)-butyl]-indol-5-carbonsäureamid, F. 141° (Z);
3-[4-(4-(4-Acetamido-phenyl)-piperazino)-butyl]-indol-5-carbonsäureamid, F. 230° (Z).

Die nachstehenden Beispiele betreffen pharmazeutische Zubereitungen, die Amine der Formel I oder ihre Säureadditionssalze enthalten:

### Beispiel A: Tabletten

Ein Gemisch von 1 kg 3-[4-(4-Phenyl-piperazino)-butyl]-indol-5-carbonsäuremethylester, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

2 kg 3-[4-(4-Phenyl-piperazino)-butyl]-5-indolyl-harnstoff werden in üblicher Weise in Hartgelatinekapseln gefüllt, so daß jede Kapsel 20 mg des Wirkstoffs enthält.

### Beispiel D: Ampullen

Eine Lösung von 1 kg 3-[4-(4-Phenyl-piperazino)-butyl]-5-indolyl-harnstoffdihydrat in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche

1. Indolderivate der Formel I 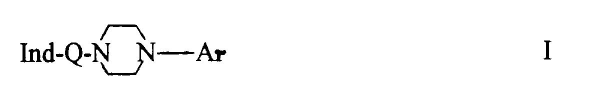 worin
Ind einen durch CN, CO-R¹, CO-NR³R⁴ oder NHR² substituierten Indol-3-ylrest,
R¹ NH₂, NHA, NA₂, NHCₙH₂ₙNA₂, NHCₙH₂ₙHet oder NHCₙH₂ₙOA,
R² CO-NH₂, CO-NHA oder CO-NA₂,
R³ und R⁴ zusammen eine Alkylengruppe mit 3-7 C-Atomen, die durch O oder NR⁵ unterbrochen und/oder durch O, NA₂, NHCOA, COOA, CONH₂, Ar oder Het substituiert sein und/oder eine zusätzliche Doppelbindung enthalten kann,
R⁵ H, A, Ar, Het, Ar-CO, COOA, CH₂CONH₂, CH₂CONA₂ oder CHO
Q C₄H₈
n 1, 2, 3, 4, 5 oder 6,
A Alkyl mit 1-6 C-Atomen,
Ar einen unsubstituierten oder einen ein-, zwei- oder dreifach durch A, F, Cl, Br, I, CN, OH, OA, NHCOA und/oder CF₃ oder durch eine Methylendioxygruppe substituierten Phenylrest und
Het einen gesättigten oder ungesättigten 5- oder 6-gliedrigen heterocyclischen Rest mit 1-4 N-, O- und/oder S-Atomer, der mit einem Benzolring kondensiert und/oder ein- oder zweifach durch A, Ar, -O(CH₂)₂O-, Carbonylsauerstoff, oder einen weiteren Rest Het substituiert sein kann,
bedeuten,
sowie deren Salze.

2. a) 3-[4-(4-p-Methoxyphenyl-piperazino)-butyl]-indol-5-carbonsäureamid;
b) 3-[4-(4-p-Methoxyphenyl-piperazino)-butyl]-5-cyan-indol;
c) 3-[4-(4-o-Methoxyphenyl-piperazino)-butyl]-indol-5-carbonsäureamid;
d) 3-[4-(4-Phenyl-piperazino)-butyl]-indol-5-carbonsäureamid;
e) 3-[4-(4-(3,4-Methylendioxy-phenyl)-piperazino)-butyl]-indol-5-carbonsäureamid
sowie
die Säureadditionssalze der genannten Verbindungen.

3. Verfahren zur Herstellung von Indolderivaten der Formel I nach Anspruch 1 sowie von deren Salzen, dadurch gekennzeichnet, daß man eine Verbindung der Formel II
Ind-Q-X¹ II
worin
X¹ X oder NH₂ und
X Cl, Br, J, OH oder eine reaktionsfähig funktionell abgewandelt OH-Gruppe bedeuten und
Ind und Q die angegebenen Bedeutungen haben,
mit einer Verbindung der Formel III
X²-CH₂-CH₂NAr-CH₂-CH₂X³ III
worin
X² und X³ gleich oder verschieden sein können und, falls X¹ = NH₂ ist, jeweils X, andernfalls zusammen NH bedeuten und
Ar die angegebene Bedeutung hat, umsetzt
oder daß man eine Verbindung der Formel IV
Ind-Q-N(CH₂-CH₂-X)₂ IV
worin
X, Q and Ind die angegebenen Bedeutungen haben, mit einer Verbindung der Formel V
Ar-NH₂ V
worin
Ar die angegebene Bedeutung hat,
umsetzt
oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en enthält, mit einem reduzierenden Mittel behandelt
oder daß man eine sonst der Formel I entsprechende Verbindung, die jedoch an Stelle eines oder mehrerer Wasserstoffatome eine oder mehrere solvolysierbare Gruppe(n) enthält, mit einem solvolysierenden Mittel behandelt
und/oder daß man gegebenenfalls eine OA-Gruppe unter Bildung einer OH-Gruppe spaltet und/oder eine Gruppe Ind und/oder eine Gruppe Ar in eine andere Gruppe Ind und/oder Ar umwandelt und/oder daß man eine erhaltene Base oder Säure der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen. flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

## Claims

1. Indole derivatives of the formula I 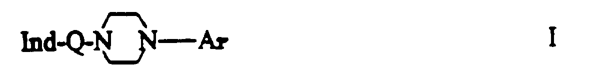 in which
Ind is an indol-3-yl radical substituted by CN, CO-R¹, CO-NR³R⁴ or NHR²,
R¹ is NH₂, NHA, NA₂, NHCₙH₂ₙNA₂, NHCₙH₂ₙHet or NHCₙH₂ₙOA,
R² is CO-NH₂, CO-NHA or CO-NA₂,
R³ and R⁴ together are an alkylene group having 3-7 C atoms, which are interrupted by O or NR⁵ and/or substituted by O, NA₂, NHCOA, COOA, CONH₂, Ar or Het and/or can contain an additional double bond,
R⁵ is H, A, Ar, Het, Ar-CO, COOA, CH₂CONH₂, CH₂CONHA, CH₂CONA₂ or CHO,
Q is C₄H₈,
n is 1,2,3,4,5 or 6,
A is alkyl with 1-6 C atoms,
Ar is a phenyl radical which is unsubstituted or mono-, di- or trisubstituted by A, F, Cl, Br, I, CN, OH, OA, NHCOA and/or CF₃ or by a methylenedioxy group and
Het is a saturated or unsaturated 5- or 6-membered heterocyclic radical having 1-4 N, O and/or S atoms, which can be fused to a benzene ring and/or mono- or disubstituted by A, Ar, -O(CH₂)₂O-, carbonyl oxygen or a further radical Het,
and their salts.

2. a) 3-[4-(4-p-Methoxyphenylpiperazino)butyl]indole-5-carboxamide;
b) 3-[4-(4-p-methoxyphenylpiperazino)butyl]-5-cyanoindole;
c) 3-[4-(4-o-methoxyphenylpiperazino)butyl]indole-5-carboxamide;
d) 3-[4-(4-phenylpiperazino)butyl]indole-5-carboxamide;
e) 3-[4-(4-(3,4-methylenedioxyphenyl)piperazino)-butyl]indole-5-carboxamide;
and
the acid addition salts of the compounds mentioned.

3. Process for the preparation of indole derivatives of the formula I according to Claim 1 and of their salts, characterized in that a compound of the formula II
Ind-Q-X¹ II
in which
X¹ is X or NH₂ and
X is Cl, Br, I, OH or a reactive functionally modified OH group and
Ind and Q have the meanings indicated, is reacted with a compound of the formula III
X²-CH₂-CH₂NAr-CH₂-CH₂X³ III III
in which
X² and X³ can be identical or different and, if X¹ = NH₂, are each X, otherwise are together NH and
Ar has the meaning indicated,
or in that a compound of the formula IV
Ind-Q-N(CH₂-CH₂-X)₂ IV IV
in which
X, Q and Ind have the meanings indicated, is reacted with a compound of the formula V
Ar-NH₂ V
in which
Ar has the meaning indicated,
or in that a compound otherwise corresponding to the formula I, but which instead of one or more hydrogen atoms contains one or more reducible group(s) and/or one or more additional C-C and/or C-N bond(s), is treated with a reducing agent
or in that a compound otherwise corresponding to the formula I, but which instead of one or more hydrogen atoms contains one or more solvolysable group(s), is treated with a solvolysing agent
and/or in that, if appropriate, an OA group is cleaved with formation of an OH group and/or a group Ind and/or a group Ar is converted into another group Ind and/or Ar and/or in that a base or acid of the formula I obtained is converted into one of its salts by treating with an acid or base.

4. Process for the production of pharmaceutical preparations, characterized in that a compound of the formula I and/or one of its physiologically acceptable salts is brought into a suitable dose form together with at least one solid, liquid or semi-liquid excipient or auxiliary.

5. Pharmaceutical preparation, characterized in that it contains at least one compound of the general formula I and/or one of its physiologically acceptable salts.

6. Use of the compounds of the formula I according to Patent Claim 1 or of their physiologically acceptable salts for the production of a medicament.

## Revendications

1. Dérivés d'indole de formule I 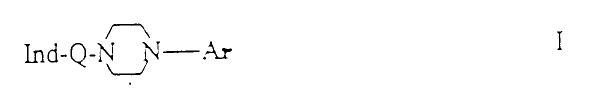 dans laquelle
Ind représente un radical indol-3-yle substitué par CN, CO-R¹, CO-NR³R⁴ ou NHR²,
R¹ représente NH₂, NHA, NA₂, NHCₙH₂ₙNA₂, NHCₙH₂ₙHet ou NHCₙH₂ₙOA,
R² représente CO-NH₂, CO-NHA ou CO-NA₂,
R³ et R⁴ représentent ensemble un groupe alkylène en C₃ à C₇, qui peut être interrompu par O ou NR₅ et/ou substitué par O, NA₂, NHCOA, COOA, CONH₂, Ar ou Het et/ou peut contenir une double liaison additionnelle,
R⁵ représente H, A, Ar, Het, Ar-CO, COOA, CH₂CONH₂, CH₂CONA₂ ou CHO,
Q représente C₄H₈,
n vaut 1, 2, 3, 4, 5 ou 6,
A représente un alkyle en C₁ à C₆,
Ar représente un radical phényle non substitué ou mono-, di- ou trisubstitué par A, F, Cl, Br, I, CN, OH, OA, NHCOA et/ou CF₃ ou par un groupe méthylènedioxy et
Het représente un radical hétérocyclique à 5 ou 6 chaînons saturé ou insaturé avec de 1 à 4 atomes de N, O et/ou S, qui peut être condensé avec un noyau benzène et/ou mono- ou disubstitué par A, Ar,
-O(CH₂)₂O, un oxygène carboxylique, ou un autre radical Het, ainsi que leurs sels.

2. a) amide de l'acide 3-[4-(4-p-méthoxyphényl-pipérazino)-butyl]-indole-5-carboxylique ;
b) 3-[4-(4-p-méthoxyphényl-pipérazino)-butyl]-5-cyano-indole ;
c) amide de l'acide 3-[4-(4-o-méthoxyphényl-pipérazino)-butyl]-indole-5-carboxylique ;
d) amide de l'acide 3-[4-(4-phényl-pipérazino)-butyl]-indole-5-carboxylique ;
e) amide de l'acide 3-[4-(4-(3,4-méthylènedioxy-phényl)-pipérazino)-butyl]-indole-5-carboxylique
ainsi que les sels d'addition d'acides des composés mentionnes.

3. Procédé de préparation de dérivés indole de formule I selon la revendication 1 ainsi que de leurs sels, caractérisé en ce qu'on fait réagir un composé ce formule II
Ind-Q-X¹ II
dans laquelle
X¹ représente X ou NH₂ et
X représente Cl, Br, I, OH ou un groupe OH fonctionnellement modifié réactif et
Ind et Q ont les significations indiquées,
avec un composé de formule III
X²-CH₂-CH₂NAr-CH₂-CH₂X³ III
dans laquelle
X² et X³ peuvent être identiques ou différents et, si
X¹ = NH₂, représentent chacun X, ou sinon, ensemble, NH et
Ar a la signification indiquée,
ou en ce qu'on fait réagir un composé de formule IV
Ind-Q-N-(CH₂-CH₂-X)₂ IV
dans laquelle
X, Q et Ind ont les significations indiquées, avec un composé de formule V
Ar-NH₂ V
dans laquelle
Ar a la signification indiquée,
ou en ce qu'on traite avec un agent réducteur un composé correspondant sinon à la formule I, mais qui contient à la place d'un ou plusieurs atomes d'hydrogène un ou plusieurs groupes réductibles et/ou une ou plusieurs liaison C-C et/ou C-N supplémentaires,
ou en ce qu'on traite avec un agent de solvolyse un composé correspondant sinon à la formule I, qui cependant contient à la place d'un ou plusieurs atomes d'hydrogène un ou plusieurs groupes solvolysables,
et/ou en ce que le cas échéant on sépare un groupe OA avec formation d'un groupe OH et/ou en ce qu'on transforme un groupe Ind et/ou un groupe Ar en un autre groupe Ind et/ou Ar, et/ou en ce qu'on transforme une base ou un acide de formule I obtenu(e) par traitement avec un acide ou une base en un de leurs sels.

4. Procédé de préparation de préparations pharmaceutiques, caractérise en ce qu'on met sous une forme posologique appropriée un composé de formule I et/ou un de ses sels physiologiquement acceptables avec au moins un support ou additif solide, liquide ou semi-liquide.

5. Préparation pharmaceutique caractérisée en ce qu'elle contient au moins un composé de formule générale I et/ou un de ses sels physiologiquement acceptables.

6. Utilisation de composés de formule I selon la revendication 1 ou de leurs sels physiologiquement acceptables pour la préparation d'un médicament.
